# FASCICULE DE BREVET EUROPEEN

(11) **EP 3 230 263 B1**
(45) Date de publication et mention de la délivrance du brevet: **19.09.2018**
(21) Numéro de dépôt: 15805177.1
(22) Date de dépôt: 07.12.2015
(51) Int. Cl.: C07C 303/00, C07C 303/14, C07C 303/32, C07C 303/38, C07C 309/00, C07C 309/06, C07C 311/48, C07C 313/04

(54) **PROCEDE DE PREPARATION DE DERIVES OXYSULFURES ET FLUORES EN MILIEU LIQUIDE IONIQUE**
VERFAHREN ZUR HERSTELLUNG VON OXYSULFIDISCHEN UND FLUORIERTEN DERIVATEN IN EINEM IONISCHEN FLÜSSIGMEDIUM
METHOD FOR PRODUCING OXYSULPHIDIC AND FLUORINATED DERIVATIVES IN AN IONIC LIQUID MEDIUM

(30) Priorité: 09.12.2014 FR 1462099
(43) Date de publication de la demande: 18.10.2017
(73) Titulaire: Rhodia Operations, 75009 Paris (FR)
(72) Inventeur: METZ, François, 69540 Irigny (FR)
(74) Mandataire: Menville, Laure
(86) Numéro de dépôt international: PCT/EP2015/078758
(87) Numéro de publication internationale: WO 2016/091772

(56) Documents cités:
- EP-A1- 0 735 023
- WO-A1-2007/128893

## Description

La présente invention a pour objet un nouveau procédé de préparation de dérivés oxysulfurés et fluorés par une réaction de sulfination conduite dans un milieu liquide ionique remplissant la double fonction de solvant et réactif.

L'invention vise plus particulièrement la préparation de sels d'acides perfluoroalcane sulfiniques et sulfoniques, et préférentiellement des sels d'acide trifluorométhanesulfinique et trifluorométhanesulfonique.

Les acides perhalogénoalcanesulfoniques, et plus particulièrement l'acide trifluorométhanesulfonique, plus connu sous l'appellation « acide triflique », sont utilisés comme catalyseurs ou comme intermédiaires en synthèse organique.

Une voie actuelle de synthèse industrielle de l'acide trifluorométhanesulfonique met en oeuvre deux principales étapes. Dans un premier temps, un sel alcalin, généralement de potassium, d'acide trifluorométhanesulfinique est synthétisé par réaction de sulfination à partir d'un sel d'acide trifluorométhanecarboxylique, dans un solvant organique aprotique, typiquement le N,N-diméthylformamide (DMF). Dans un second temps, le sel d'acide trifluorométhanesulfinique est oxydé en milieu aqueux, généralement par l'eau oxygénée, en un sel d'acide trifluorométhanesulfonique, qui conduira, après acidification, à l'acide triflique. La préparation d'acides perfluorométhanesulfiniques sous forme salifiée est par exemple décrite dans les documents EP 0 735 023 et WO 2007/128893.

Même si ce procédé donne globalement satisfaction, certains éléments pourraient être améliorés. D'une part, on souhaiterait limiter les étapes de basculement milieu organique/milieu aqueux entre les réactions de sulfination et d'oxydation, car ces étapes de basculement peuvent être complexes à opérer. En outre, la présence d'eau lors de l'étape d'acidification est un inconvénient, et on doit mettre en oeuvre des moyens pour capter cette eau résiduelle, typiquement de l'ajout d'anhydride sulfurique (SO₃). L'ajout d'anhydride sulfurique pour capter l'eau résiduelle se traduit malheureusement par la génération d'une grande quantité d'effluents sulfuriques.

La présente invention vise à proposer un nouveau procédé de préparation de dérivés oxysulfurés et fluorés, en particulier utile pour la synthèse de l'acide trifluorométhanesulfonique, et ne présentant pas les inconvénients évoqués précédemment.

Plus précisément, la présente invention concerne, selon un premier de ses aspects, un procédé de préparation d'un dérivé oxysulfuré et fluoré sous forme de sel de formule (II)

Ea-SOO⁻Q⁺ (II)

comprenant la mise en présence d'un composé liquide ionique de formule (I) à l'état liquide

Ea-COO⁻Q⁺ (I)

- Ea représentant l'atome de fluor ou un groupe ayant de 1 à 10 atomes de carbone choisi parmi les fluoroalkyles, les perfluoroalkyles et les fluoroalkényles ; et
- Q⁺ représentant un cation onium,
avec un oxyde de soufre,
ledit composé liquide ionique de formule (I) représentant au moins 50 % en poids du milieu réactionnel liquide initial.

De manière surprenante, les inventeurs ont montré que la réaction de sulfination pouvait être conduite dans un milieu liquide ionique remplissant la double fonction de solvant et réactif, avec des performances en termes de cinétique et de sélectivité au moins identiques à celles d'une sulfination en milieu solvant organique, typiquement le DMF.

De manière avantageuse, le procédé selon l'invention permet ainsi de s'affranchir de la mise en oeuvre d'un solvant annexe, typiquement le DMF, pour réaliser la sulfination.

Egalement, comme développé dans la suite du texte, il est possible d'opérer selon l'invention l'oxydation, pour accéder par exemple à un sel d'acide trifluorométhane sulfonique, à partir du mélange du dérivé oxysulfuré et fluoré Ea-SOO⁻Q⁺ (II) issu de la sulfination et du composé liquide ionique Ea-COO⁻Q⁺ (I) non réagi, liquide à la température de la réaction d'oxydation et servant à la fois de réactif et de solvant de la réaction d'oxydation.

Ainsi, l'oxydation selon l'invention peut être opérée sans nécessiter l'ajout de solvant aqueux.

De manière avantageuse, les étapes de sulfination et d'oxydation selon l'invention peuvent être réalisées successivement et sans étape intermédiaire de basculement de solvants (milieu organique/milieu aqueux), en particulier au sein du même réacteur.

Le procédé de l'invention permet avantageusement un gain de temps et d'énergie, et donc une diminution du coût de revient, du fait de la réduction du nombre d'étapes nécessaires à l'obtention du sel de trifluorométhanesulfonate (et de l'acide triflique) par exemple.

Par ailleurs, l'enchaînement des étapes de sulfination et d'oxydation selon l'invention permet de minimiser la dégradation du flux réactionnel issu de la sulfination, qui peut avoir lieu lors du basculement de solvants.

Ainsi, la mise en oeuvre du procédé de l'invention permet d'améliorer le rendement global de la préparation du sel de trifluorométhanesulfonate (et de l'acide triflique).

Enfin, le procédé de l'invention, en s'affranchissant de la mise en oeuvre de solvant aqueux pour l'oxydation, permet d'accéder à l'acide triflique de qualité électronique, présentant une faible teneur en sulfates, voire étant exempt de sulfates.

Bien entendu, la synthèse de l'acide triflique est donnée à titre illustratif, le procédé de l'invention n'étant nullement limité à la seule synthèse d'un sel de trifluorométhanesulfonate et à celle de l'acide triflique.

D'autres caractéristiques, variantes et avantages du procédé selon l'invention ressortiront mieux à la lecture de la description et des exemples qui vont suivre, donnés à titre illustratif et non limitatif de l'invention.

Dans la suite du texte, les expressions « compris entre ... et ... », « allant de ... à ... » et «variant de ... à ... » sont équivalentes et entendent signifier que les bornes sont incluses, sauf mention contraire.

Comme précisé précédemment, le procédé de préparation d'un dérivé oxysulfuré et fluoré sous forme de sel de formule Ea-SOO⁻Q⁺ (II) selon l'invention met en jeu une réaction de sulfination d'un composé liquide ionique de formule Ea-COO⁻Q⁺ (I) avec un oxyde de soufre. La mise en présence du composé liquide ionique de formule (I) avec un oxyde de soufre est mise en oeuvre dans des conditions propices à la formation du dérivé de formule (II).

Le composé liquide ionique de formule (I) selon l'invention joue à la fois le rôle de réactif et de solvant de la réaction de sulfination.

Le milieu réactionnel initial de la réaction de sulfination selon l'invention comprend ledit composé liquide ionique de formule (I) à l'état liquide à la température de la réaction et éventuellement l'oxyde de soufre selon si la réaction est opérée en mode discontinu (batch pur) ou en mode semi-continue (semi-batch) ou continu.

Par « milieu réactionnel », on entend, au sens de l'invention, le milieu dans lequel a lieu la réaction chimique concernée, dans le cas présent la réaction de sulfination. Le milieu réactionnel comprend généralement le solvant de réaction (dans le cas présent, le composé liquide ionique qui est également un des réactifs) et, en fonction de l'avancement de la réaction, les réactifs et/ou les produits de la réaction. Il peut comprendre en outre des additifs et des impuretés.

Le milieu réactionnel dit « initial » est le milieu mis en oeuvre dans le réacteur avant réaction et formation du produit recherché. Le milieu réactionnel initial peut comprendre un des réactifs (par exemple, en mode semi-continu) ou l'ensemble des réactifs (par exemple, en mode discontinu).

Dans le cas de la présente réaction de sulfination, le composé liquide ionique de formule (I), jouant la double fonction de solvant et réactif, représente au moins 50 % en poids du milieu réactionnel liquide initial.

Dans le cas particulier de la mise en oeuvre de la réaction de sulfination en mode discontinu (batch pur), le milieu réactionnel initial comprend le composé liquide ionique et l'intégralité de la quantité d'oxyde de soufre. Le composé liquide ionique de formule (I) peut représenter de 50 à 95 % en poids du milieu réactionnel initial.

Dans le cas particulier de la mise en oeuvre de la réaction de sulfination en mode semi-continu (semi-batch), l'oxyde de soufre peut être ajouté en continu. Le composé liquide ionique de formule (I) peut ainsi représenter de 50 à 100 % en poids du milieu réactionnel initial.

De préférence, le composé liquide ionique de formule (I) représente au moins 60 % en poids, en particulier au moins 70 % en poids et plus particulièrement au moins 80 % en poids du milieu réactionnel initial.

De préférence, le milieu réactionnel de la réaction de sulfination selon l'invention est exempt de solvant autre que ledit composé liquide ionique.

Par « solvant », on entend désigner au sens de l'invention un composé liquide à sa température d'utilisation, apte, de par sa teneur dans le milieu réactionnel, à solubiliser un réactif.

En particulier, le milieu réactionnel de la réaction est exempt de solvant organique, notamment de solvant organique de type amide tel que le N,N-diméthylformamide (DMF), la N-méthylpyrrolidone (NMP) ou le N,N-diméthylacétamide (DMAC).

Selon un mode de réalisation particulièrement préféré, le milieu réactionnel liquide initial de la réaction de sulfination selon l'invention est formé du composé liquide ionique de formule (I) à l'état liquide à la température de la réaction et éventuellement d'un oxyde de soufre selon si la réaction est opérée en mode discontinu (batch pur) ou en mode semi-continu (semi-batch) ou continu.

D'une manière générale, les liquides ioniques sont des liquides qui ne contiennent essentiellement que des ions. L'expression « liquide ionique » désigne un sel ayant un point de fusion inférieur ou égal à 100 °C.

Les liquides ioniques englobent notamment un groupe de composé ioniques liquides à température ambiante (20 °C) et qui sont appelés « liquides ioniques liquides à température ambiante » (RTIL pour l'expression en langue anglaise « Room-Temperature Ionic Liquids »).

Ainsi, au sens de l'invention, conformément à la définition communément reconnue, le composé liquide ionique de formule (I) mis en oeuvre selon le procédé de l'invention possède un point de fusion inférieur à 100 °C.

En particulier, le composé liquide ionique de formule (I) possède un point de fusion inférieur à 80 °C, en particulier inférieur à 60 °C et plus particulièrement inférieur à 40 °C.

Selon un mode de réalisation particulier, le composé liquide ionique selon l'invention possède un point de fusion inférieur à 20 °C (liquide ionique liquide à température ambiante).

Le composé liquide ionique de formule (I) selon l'invention est ainsi à l'état liquide dans les conditions de la réaction de sulfination.

Comme indiqué précédemment, Q⁺ représente un cation onium.

Les cations onium sont des cations formés par les éléments des colonnes V B et VI B (tels que définis par l'ancien système IUPAC européen selon le tableau périodique des éléments, encore appelé table de Mendeleïev) avec trois ou quatre chaînes hydrocarbonées. La colonne V B comprend les atomes N, P, As, Sb et Bi. La colonne VI B comprend les atones O, S, Se, Te et Po. Le cation onium peut en particulier être un cation formé par un atome choisi dans le groupe constitué par N, P, O et S, plus préférentiellement N et P, avec trois ou quatre chaînes hydrocarbonées.

Le cation onium Q⁺ peut être choisi parmi :
- Les cations onium hétérocycliques ; en particuliers ceux choisis dans le groupe constitué par 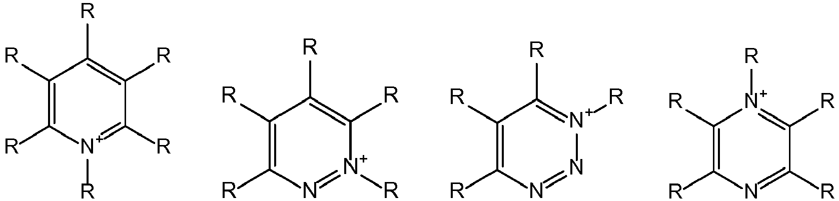 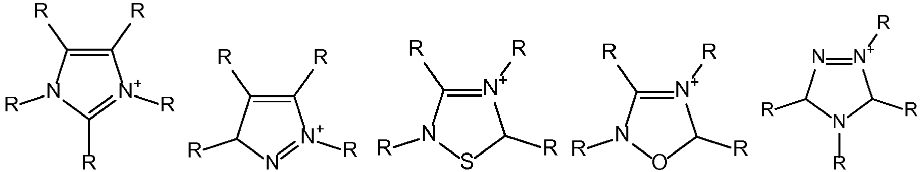
- Les cations onium cycliques insaturés ; en particuliers ceux choisis dans le groupe constitué par 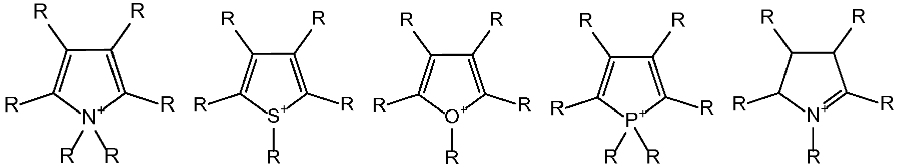
- Les cations onium cycliques saturés ; en particuliers ceux choisis dans le groupe constitué par 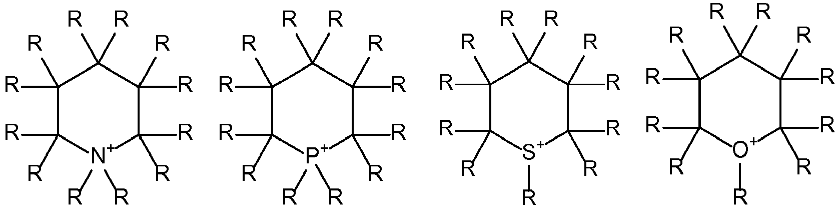 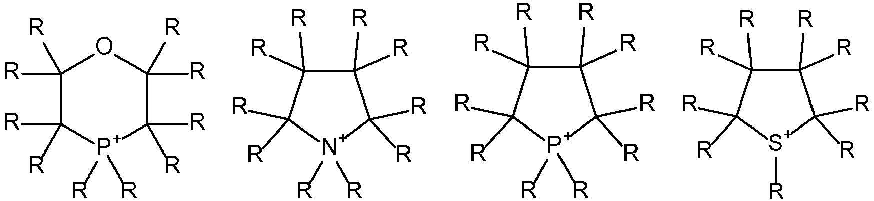 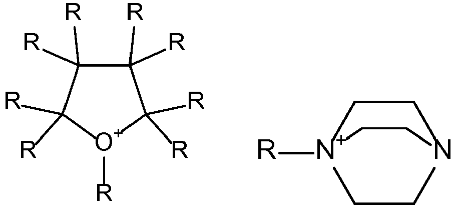
- Les cations onium non cycliques ; en particuliers ceux de formule générale ⁺L-R'ₛ, dans laquelle L représente un atome choisi dans le groupe constitué par N, P, O et S, plus préférentiellement N et P, s représente le nombre de groupe R' choisi parmi 2, 3 ou 4 selon la valence de l'élément L, chaque R' représente indépendamment un atome d'hydrogène ou un groupe alkyle en C₁ à C₈, et la liaison entre L⁺ et R' peut être une liaison simple ou une liaison double.

Dans les formules ci-dessus, chaque symbole « R » représente, indépendamment les uns des autres, un atome d'hydrogène ou un groupe organique ou peuvent être liés les uns avec les autres. De façon préférée, chaque symbole « R » peut représenter dans le formules ci-dessus, indépendamment les uns des autres, un atome d'hydrogène ou un groupe hydrocarboné en C₁ à C₁₈, linéaire, branché ou cyclique, saturé ou insaturé, éventuellement substitué une ou plusieurs fois par un atome d'halogène, un groupe amino, une groupe imino, une groupe amide, une groupe éther, un groupe ester, un groupe hydroxyle, un groupe carboxyle, un groupe carbamoyle, un groupe cyano, un groupe sulfone ou un groupe sulfite.

Le cation onium Q⁺ peut être plus particulièrement choisi parmi les cations ammonium, phosphonium, pyridinium, pyrazolinium, imidazolium, arsénium, ammonium quaternaire et phosphonium quaternaire.

Les cations ammonium quaternaire ou phosphonium quaternaire peuvent être plus préférentiellement choisis parmi les tétraalkylammonium ou tétraalkylphosphonium, les triakylbenzylammonium ou triakylbenzylphosphonium, les tétraarylammonium ou tétraarylphosphonium, dont les groupes alkyles, identiques ou différents, représentent une chaîne alkyle linéaire ou ramifiée, ayant de 4 à 12 atomes de carbone, de préférence de 4 à 6 atomes de carbone, et les groupes aryles, identiques ou différents, représentent un groupe phényle ou naphtalényle.

Selon un mode de réalisation particulier, Q⁺ représente un cation phosphonium quaternaire ou ammonium quaternaire.

Selon un mode de réalisation particulièrement préféré, Q⁺ représente un cation phosphonium quaternaire, en particulier un cation tétraalkylphosphonium, et plus particulièrement le cation tétrabutylphosphonium (PBu₄).

Selon un autre mode de réalisation préféré, Q⁺ représente un cation ammonium quaternaire, en particulier choisi dans le groupe constitué par le tétraéthylammonium, le tétrapropylammonium, le tétrabutylammonium, le triméthylbenzylammonium, le méthyltributylammonium et l'Aliquat 336 (mélange de composés méthyltri(alkyl en C₈ à C₁₀)ammonium).

Selon un autre mode de réalisation préféré, Q⁺ représente un cation pyridinium, en particulier le N-méthylpyridinium.

Comme indiqué précédemment, le groupe Ea peut représenter l'atome de fluor ou un groupe ayant de 1 à 10 atomes de carbone choisi parmi les fluoroalkyles, les perfluoroalkyles et les fluoroalkényles.

Dans le cadre de l'invention, on entend par :
- alkyle, une chaîne hydrocarbonée linéaire ou ramifiée comportant de préférence de 1 à 10 atomes de carbone, en particulier de 1 à 4 atomes de carbone ;
- fluoroalkyle, un groupe formé d'une chaîne hydrocarbonée linéaire ou ramifiée en C₁-C₁₀ comportant au moins un atome de fluor ;
- perfluoroalkyle, un groupe formé d'une chaîne linéaire ou ramifiée en C₁-C₁₀ comprenant seulement des atomes de fluor, en plus des atomes de carbone, et dépourvue d'atome d'hydrogène ;
- fluoroalkényle, un groupe formé d'une chaîne hydrocarbonée en C₁-C₁₀, linéaire ou ramifiée, comportant au moins un atome de fluor, et comprenant au moins une double liaison.

De manière préférée, le groupe Ea est choisi parmi l'atome de fluor et un groupe ayant de 1 à 5 atomes de carbone choisi parmi les fluoroalkyles, les perfluoroalkyles et les fluoroalkényles.

Selon un mode de réalisation particulièrement préféré, le groupe Ea dans le composé de formule (I) est choisi parmi l'atome de fluor, le radical CH₂F, le radical CHF₂, le radical C₂F₅ et le radical CF₃. Il en résulte ainsi respectivement la préparation selon le procédé de l'invention de F-SOO⁻Q⁺; de CH₂F-SOO⁻Q⁺, de CHF₂-SOO⁻Q⁺, de C₂F₅-SOO⁻Q⁺ et de CF₃-SOO⁻Q⁺, où Q⁺ est tel que défini précédemment.

Selon un mode de réalisation particulier, Ea représente le radical CF₃.

Il est entendu que les définitions précitées respectivement pour le cation onium Q⁺ et le groupe Ea peuvent être combinées.

Ainsi, selon une variante de réalisation, le procédé de l'invention met en oeuvre un composé liquide ionique de formule Ea-COO⁻Q⁺ (I), dans laquelle :
- Ea est choisi parmi l'atome de fluor, le radical CH₂F, le radical CHF₂, le radical C₂F₅ et le radical CF₃ ; en particulier Ea est le radical CF₃ ; et
- Q⁺ représente un cation phosphonium quaternaire, en particulier le cation tétrabutylphosphonium (PBu₄).

Le composé liquide ionique de formule Ea-COO⁻Q⁺ (I) selon l'invention peut être préparé, préalablement à sa mise en oeuvre dans la réaction de sulfination selon le procédé de l'invention.

Selon une première variante de réalisation, il peut être obtenu par réaction, de préférence en présence d'un solvant aqueux, d'un sel alcalin de formule Ea-COO⁻R⁺ avec R⁺ représentant un cation de métal alcalin, par exemple de potassium, avec un sel d'onium Q⁺, suivie, de préférence, de l'élimination du solvant aqueux.

Selon une autre variante de réalisation, le composé liquide ionique de formule (I) selon l'invention peut être obtenu par réaction, dans un solvant organique, d'un acide fluorocarboxylique de formule Ea-COOH ou de son sel Ea-COO⁻ avec un sel d'onium Q⁺, suivie, de préférence, de la récupération du composé liquide ionique par extraction du solvant organique.

Le sel d'onium Q⁺ pour la préparation du composé liquide ionique peut être plus particulièrement un halogénure d'onium, en particulier un chlorure d'onium.

Le composé liquide ionique de formule (I) mis en oeuvre dans le procédé de l'invention peut être totalement ou en partie un composé recyclé, qui peut être obtenu, par exemple, par séparation à l'issue de la réaction de sulfination ou qui peut provenir d'une étape ultérieure de synthèse, par exemple par séparation à l'issue de la préparation d'un sel d'acide fluorosulfonique par oxydation.

L'oxyde de soufre peut être plus particulièrement du dioxyde de soufre.

Il peut être mis en oeuvre sous forme gazeuse.

L'homme du métier est à même d'adapter les conditions de mise en oeuvre, notamment en termes de température et de durée, de la réaction de sulfination selon l'invention, pour conduire au dérivé oxysulfuré et fluoré sous forme de sel de formule Ea-SOO⁻Q⁺ (II).

Le composé liquide ionique de formule (I) et l'oxyde de soufre peuvent être mis en oeuvre dans un rapport molaire oxyde de soufre/composé de formule (I) compris entre 0,5 et 2, en particulier entre 0,6 et 1,2.

La mise en contact de l'oxyde de soufre, en particulier du dioxyde de soufre, avec le composé liquide ionique de formule (I) peut être effectuée de manière continue, semi-continue (semi-batch) ou discontinue (batch).

Le procédé selon l'invention peut être opéré dans un appareillage permettant une mise en oeuvre en semi-continu, en continu ou discontinu, par exemple dans un réacteur parfaitement agité, une cascade de réacteurs parfaitement agités avantageusement équipés d'une double enveloppe ou un réacteur tubulaire équipé d'une double enveloppe dans lequel circule un fluide caloporteur.

Selon un mode de réalisation particulièrement préféré, la mise en contact de l'oxyde de soufre, en particulier du dioxyde de soufre, avec le composé liquide ionique de formule (I) est réalisée de manière semi-continue (semi-batch).

De préférence, l'oxyde de soufre, en particulier le dioxyde de soufre, est ajouté progressivement dans un milieu liquide, préparé préalablement, formé du composé liquide ionique de formule (I) à l'état liquide.

La réaction de sulfination selon le procédé de l'invention peut être opérée en portant le milieu réactionnel à une température comprise entre 100 °C et 200 °C, en particulier entre 120 °C et 150 °C.

La durée du chauffage peut être ajustée en fonction de la température de réaction choisie. Elle peut être d'au moins une heure, en particulier être comprise entre 2 heures et 5 heures.

La réaction de sulfination est avantageusement mise en oeuvre sous pression atmosphérique. Des pressions plus élevées peuvent être également utilisées. Ainsi, peut convenir une pression totale absolue choisie entre 1 et 20 bar, de préférence entre 1 et 3 bar. Selon un autre mode de réalisation, la réaction peut être mise en oeuvre à une pression inférieure à la pression atmosphérique. La pression totale absolue peut être comprise entre 1 mbar et 999 mbar, en particulier entre 500 mbar et 950 mbar, et plus particulièrement entre 800 mbar et 900 mbar.

Selon un mode de réalisation en continu, le temps de séjour moyen qui est défini comme le rapport entre le volume de la masse réactionnelle et le débit d'alimentation, se situe plus particulièrement entre 30 minutes et 10 heures, en particulier entre 2 heures et 4 heures.

De manière avantageuse, l'avancement de la réaction de sulfination peut être suivi par une méthode analytique.

L'avancement de la réaction de sulfination, par exemple l'évolution de la concentration en dérivé oxysulfuré et fluoré formé de formule (II), peut être suivi en ligne (*via* une boucle d'échantillonnage par exemple) ou *in situ* par spectrométrie RAMAN, par spectrométrie proche infrarouge ou par spectroscopie UV, de préférence par spectrométrie RAMAN.

Dans le cadre du suivi de l'état d'avancement de la réaction par spectrométrie RAMAN, le réacteur au sein duquel a lieu la réaction d'oxydation, peut être équipé d'une sonde Raman, reliée par une fibre optique au spectromètre Raman, ladite sonde permettant par exemple de suivre dans le milieu la concentration en composé de formule (II).

Il est préférable de ne pas chercher à convertir complètement le composé liquide ionique de formule (I) de départ.

L'avancement de la réaction de sulfination peut être contrôlé par le taux de conversion du composé de formule (I) qui désigne le rapport entre la quantité molaire de composé de formule (I) consommé au cours de la réaction sur la quantité totale de composé de formule (I) dans le milieu réactionnel initial. Ce taux peut être aisément calculé après dosage dudit composé de formule (I) restant dans le milieu réactionnel.

D'une manière générale, la réaction de sulfination est conduite jusqu'à un taux de conversion dudit composé de formule (I) allant de 10 % à 90 %, en particulier de 30 % à 70 % et plus particulièrement de 50 % à 60 %.

A l'issue de la réaction de sulfination selon l'invention, le milieu réactionnel comprend ainsi un mélange du composé liquide ionique non consommé Ea-COO⁻Q⁺ (I) et du dérivé oxysulfuré et fluoré formé Ea-SOO⁻Q⁺ (II).

Le procédé de l'invention peut être plus particulièrement mis en oeuvre pour la préparation d'un sel d'onium d'acide trifluorométhanesulfinique (CF₃SO₂⁻Q⁺ avec Q⁺ représentant un cation onium), en particulier du trifluorométhanesulfinate de tétrabutylphosphonium (CF₃SO₂PBu₄, ou triflinate de tétrabutylphosphonium).

Ce dernier pourra avantageusement être utilisé pour accéder par exemple au bis(trifluorométhanesulfonyl)imidure de lithium (CF₃SO₂)₂NLi (LiTFSI), à l'acide triflique CF₃SO₃H ou encore à l'anhydride triflique (CF₃-SO₂)₂O, comme détaillé dans la suite du texte.

Le composé oxysulfuré et fluoré de formule Ea-SOO⁻Q⁺ (II) peut être utilisé pour former, par réaction d'oxydation, un composé de formule Ea-SO₃⁻Q⁺ (III).

La présente invention concerne ainsi, selon un autre de ses aspects, un procédé de préparation d'un composé sous forme de sel de formule (III)

Ea-SO₃⁻Q⁺ (III)

- Ea représentant l'atome de fluor ou un groupe ayant de 1 à 10 atomes de carbone choisi parmi les fluoroalkyles, les perfluoroalkyles et les fluoroalkényles ; et
- Q⁺ représentant un cation onium,
comprenant les étapes consistant en :
(i) disposer d'un mélange (M), présentant une température de fusion inférieure ou égale à 100 °C, d'un composé liquide ionique de formule Ea-COO⁻Q⁺ (I) et d'un composé de formule Ea-SOO⁻Q⁺ (II) ; et
(ii) mettre en présence ledit mélange (M) à l'état liquide avec un agent oxydant pour obtenir le composé de formule (III), ledit mélange (M) représentant plus de 50 % en poids du milieu réactionnel liquide initial.

Le mélange (M) de l'étape (i) peut être le mélange liquide réactionnel directement obtenu à l'issue de l'étape de sulfination décrite précédemment, la réaction de sulfination étant plus particulièrement conduite jusqu'à un taux de conversion dudit composé liquide ionique de formule (I) allant de 10 % à 90 %, en particulier de 30 % à 70 % et plus particulièrement de 50 % à 60 %.

Selon un mode de réalisation particulièrement avantageux, les étapes de sulfination et d'oxydation peuvent être enchainées, par exemple au sein d'un même réacteur semi-batch, sans nécessiter d'opération de changement de solvant.

Le composé de formule Ea-SO₃⁻Q⁺ (III), où Ea et Q⁺ sont tels que définis précédemment, peut être ainsi préparé selon l'invention *via* les étapes suivantes:
(a) mise en contact d'un composé liquide ionique de formule Ea-COO⁻Q⁺ (I) à l'état liquide où Q représente un cation onium, avec un oxyde de soufre, pour former un composé de formule Ea-SOO⁻Q⁺ (II), ledit composé liquide ionique de formule (I) représentant au moins 50 % en poids du milieu réactionnel liquide initial ;
   la réaction de sulfination étant conduite jusqu'à un taux de conversion dudit composé de formule (I) allant de 10 à 90 %, en particulier de 30 à 70 % et plus particulièrement de 50 à 60 % ;
(b) ajout au mélange réactionnel liquide (M) des composés de formule Ea-COO⁻Q⁺ (I) et Ea-SOO⁻Q⁺ (II), obtenu à l'issue de l'étape (a) de sulfination, d'un agent oxydant, pour former un composé de formule Ea-SO₃⁻Q⁺ (III).

Le mélange (M) des composés de formule Ea-COO⁻Q⁺ (I) et Ea-SOO⁻Q⁺ (II) peut présenter plus particulièrement une température de fusion inférieure ou égale à 80 °C, en particulier inférieure ou égale à 40 °C, et de préférence inférieure ou égale à 20 °C.

Le dérivé oxysulfuré et fluoré de formule Ea-SOO⁻Q⁺ (II) et le composé liquide ionique de formule Ea-COO⁻Q⁺ (I) peuvent être présents au sein du mélange (M) dans un rapport pondéral composé (II)/composé (I) compris entre 0,2 et 4, en particulier entre 0,5 et 3.

Bien entendu, la composition du mélange (M), formé à l'issue du procédé de sulfination selon l'invention décrit précédemment, dépend du taux de conversion du composé de formule (I) à l'issue de la réaction de sulfination.

De manière avantageuse, la réaction d'oxydation selon l'invention ne nécessite pas l'ajout d'un solvant annexe.

En particulier, le milieu réactionnel de la réaction d'oxydation selon l'invention est exempt de solvant aqueux.

L'absence de solvant aqueux n'exclut pas la présence éventuelle d'eau en une faible teneur inadaptée à la solubilisation d'un réactif.

Ainsi, le milieu réactionnel de la réaction d'oxydation selon l'invention peut comprendre une teneur en eau inférieure ou égale à 20 % en poids, en particulier inférieure ou égale à 10 % en poids.

Ces faibles quantités d'eau peuvent plus particulièrement provenir de l'agent oxydant mis en oeuvre pour la réaction d'oxydation, par exemple de l'eau oxygénée, et/ou être formées par la réaction d'oxydation.

Le mélange des composés de formule Ea-COO⁻Q⁺ (I) et Ea-SOO⁻Q⁺ (II), à l'état liquide dans les conditions de la réaction d'oxydation, sert à la fois de réactif et de solvant de la réaction d'oxydation.

Le mélange, noté (M), des composés de formule Ea-COO⁻Q⁺ (I) et Ea-SOO⁻Q⁺ (II) représente ainsi au moins 50 % en poids du milieu réactionnel liquide initial.

Dans le cas particulier de la mise en oeuvre de la réaction d'oxydation en mode discontinu (batch pur), le milieu réactionnel initial comprend ledit mélange (M) et l'intégralité de la quantité d'agent oxydant mis en oeuvre pour la réaction d'oxydation. Le mélange liquide (M) selon l'invention peut représenter de 50 à 95 % en poids du milieu réactionnel initial.

Dans le cas particulier de la mise en oeuvre de la réaction d'oxydation en mode semi-continu (semi-batch), l'agent oxydant peut être ajouté progressivement. Le mélange liquide (M) selon l'invention peut représenter de 50 % à 100 % en poids du milieu réactionnel initial.

Selon un mode de réalisation particulièrement préféré, le milieu réactionnel initial de l'étape d'oxydation selon l'invention est formé dudit mélange (M) des composés de formule Ea-COO⁻Q⁺ (I) et Ea-SOO⁻Q⁺ (II), et éventuellement de l'agent oxydant, selon si la réaction d'oxydation est opérée en mode discontinu (batch pur), semi continu (semi-batch) ou continu.

L'agent oxydant peut être choisi parmi les peroxydes, les peracides et leurs sels. Par exemple, l'agent oxydant peut être choisi parmi l'eau oxygénée ; les percarbonates, notamment le percarbonate de sodium ou de potassium ; les persulfates notamment le persulfate de potassium ; l'acide persulfurique, par exemple le sel de Caro ; et les peroxydes organiques, par exemple le peroxyde d'hydrogène-urée. L'agent oxydant peut encore être l'hypochlorite de sodium.

L'agent oxydant peut être miscible ou pas dans le milieu réactionnel. Ainsi, le milieu réactionnel peut être homogène ou hétérogène.

Selon un mode de réalisation particulièrement avantageux, l'agent oxydant est anhydre.

Selon un autre mode de réalisation particulier, l'agent oxydant est l'eau oxygénée. L'eau oxygénée peut avoir une concentration dans l'eau comprise entre 10% et 80%, de préférence entre 30% et 70%.

Par ailleurs, l'agent oxydant peut être choisi parmi les agents gazeux, par exemple dans le groupe constitué par l'air, l'oxygène (O₂), l'ozone (O₃) et le protoxyde d'azote (N₂O). L'oxydation avec ces agents peut éventuellement se faire en présence de catalyseur métallique.

L'homme du métier est à même d'adapter les conditions de mise en oeuvre de la réaction d'oxydation pour conduire au composé de formule (III) souhaité.

La mise en contact du mélange liquide (M) avec l'agent oxydant peut être effectuée de manière continue, semi-continue ou discontinue. De préférence, elle est réalisée de manière semi-continue (semi-batch). Elle peut être réalisée dans un appareillage tel que décrit précédemment pour le procédé de sulfination selon l'invention.

Selon un mode de mise en oeuvre semi-continu particulièrement préféré, l'agent oxydant, par exemple l'eau oxygénée, est ajouté progressivement dans le mélange liquide (M).

La réaction d'oxydation selon le procédé de l'invention peut être opérée en portant le milieu réactionnel à une température comprise entre 20°C et la température d'ébullition du solvant organique, en particulier entre 40°C et 140°C. De manière avantageuse, l'agent oxydant peut être ajouté après avoir préalablement chauffé le mélange (M).

La durée du chauffage peut être ajustée en fonction de la température de réaction choisie. Elle peut être comprise entre 30 minutes et 24 heures, en particulier entre 1 heure et 20 heures, et plus particulièrement entre 2 heures et 7 heures.

Comme pour l'étape de sulfination décrite précédemment, l'avancement de la réaction d'oxydation peut être suivi par une méthode analytique telle que décrite précédemment, de préférence par spectrométrie RAMAN.

A l'issue de la réaction d'oxydation selon l'invention, le milieu réactionnel est formé essentiellement du mélange du composé liquide ionique de formule Ea-COO⁻Q⁺ (I) et du composé formé de formule Ea-SO₃⁻Q⁺ (III).

De manière avantageuse, le procédé d'oxydation selon l'invention est mis en oeuvre pour préparer un sel d'onium d'acide trifluorométhanesulfonique (CF₃SO₃⁻Q⁺ avec Q⁺ représentant un cation onium), en particulier du trifluorométhanesulfonate de tétrabutylphosphonium (CF₃SO₃PBu₄, ou triflate de tétrabutylphosphonium).

Ce dernier pourra avantageusement être utilisé pour accéder à l'acide triflique (CF₃SO₃H) ou encore à l'anhydride triflique ((CF₃SO₂)₂O), comme détaillé dans la suite du texte.

Les dérivés oxysulfurés et fluorés de formule Ea-SO₂⁻Q⁺ (II) obtenus selon le procédé de sulfination de l'invention décrit précédemment peuvent être avantageusement utilisés pour la synthèse de composés sulfonimides de formule (Ea-SO₂)₂NH (IV) et de leurs sels (Ea-SO₂)₂NMe (IV'), avec Me représentant un métal alcalin.

Selon un autre de ses aspects, l'invention concerne ainsi un procédé de préparation d'un composé sulfonimide (Ea-SO₂)₂NH (IV) ou un de ses sels (Ea-SO₂)₂NMe (IV'),

Ea représentant l'atome de fluor ou un groupe ayant de 1 à 10 atomes de carbone choisi parmi les fluoroalkyles, les perfluoroalkyles et les fluoroalkényles ; et
Me représentant un métal alcalin, en particulier le lithium ;
comprenant au moins les étapes suivantes :
(a1) préparation d'un dérivé oxysulfuré et fluoré de formule Ea-SO₂⁻Q⁺ (II) selon le procédé décrit ci-dessus ;
(b1) bromation, chloration ou fluoration du dérivé de formule (II) pour former un composé de formule (Ea-SO₂)X avec X représentant le brome, le chlore ou le fluor ;
(c1) ammonolyse du composé (Ea-SO₂)X à l'aide d'une amine tertiaire NR"₃ en (EaSO₂)₂NH.NR"₃, avec R", identiques ou différents, représentant un groupe alkyle, linéaire ou ramifié, ayant de 1 à 20 atomes de carbone ;
(d1) acidification de (EaSO₂)₂NH.NR"₃ pour obtenir le composé sulfonimide (Ea-SO₂)₂NH (IV) ;
et éventuellement :
(e1) neutralisation, par une base de métal alcalin Me, en particulier par un hydroxyde alcalin, du composé (Ea-SO₂)₂NH pour former le sel de formule (Ea-SO₂)₂NMe (IV') ; et éventuellement
(f1) séchage du sel (Ea-SO₂)₂NMe (IV').

L'amine tertiaire mise en oeuvre pour l'étape (c1) d'ammonolyse peut être par exemple la di-isopropyl-éthyl-amine (EDIPA).

Selon un autre de ses aspects, la présente invention a également pour objet l'enchaînement des étapes (a1) et (b1) décrites ci-dessus. Ainsi, l'invention concerne un procédé de préparation d'un composé (Ea-SO₂)X avec X représentant le chlore ou le fluor, comprenant :
- la préparation d'un dérivé oxysulfuré et fluoré de formule Ea-SO₂⁻Q⁺ (II) selon le procédé décrit ci-dessus ; et
- la bromation, la chloration ou fluoration du dérivé de formule (II) pour former un composé de formule (Ea-SO₂)X avec X représentant le brome, le chlore ou le fluor.

Selon un mode de réalisation particulièrement préféré, le dérivé oxysulfuré et fluoré de formule (II) est un sel d'onium d'acide trifluorométhanesulfinique CF₃SO₂⁻Q⁺, par exemple le trifluorométhanesulfinate de tétrabutylphosphonium (CF₃SO₂PBu₄), pour accéder, selon le procédé décrit précédemment au bis-(trifluorométhanesulfonyl)imide (CF₃SO₂)₂NH et au bis-(trifluorométhanesulfonyl)imidure de lithium (CF₃SO₂)₂NLi(LiTFSI).

Selon un autre mode de réalisation particulièrement préféré, le dérivé oxysulfuré et fluoré de formule (II) est un sel d'onium d'acide fluorosulfinique F-SO₂⁻Q⁺, par exemple le fluorosulfinate de tétrabutylphosphonium (F-SO₂PBu₄), pour accéder, selon le procédé décrit précédemment, au bis-(fluorosulfonyl)imide (F-SO₂)₂NH et au bis-(fluorosulfonyl)imidure de lithium (F-SO₂)₂NLi (LiFSI).

Les composés sulfonimides et leurs sels, préparés selon le procédé décrit ci-dessus, peuvent avantageusement être utilisés comme sels d'électrolyte, comme précurseurs d'agent antistatique ou encore comme précurseurs de tensio-actif. En particulier, lesdits composés peuvent avantageusement être employés comme électrolytes pour la fabrication de batteries, dans le domaine de l'électrochromisme, de l'électronique et de l'électrochimie. Ils sont avantageusement employés comme agents antistatiques pour la fabrication d'adhésifs sensibles à la pression (PSA : pressure sensitive adhesives). En tant qu'agents antistatiques, ils peuvent encore être employés comme composants de lubrifiants. Ils sont utilisés dans les matériaux optiques tels que les appareils électroluminescents et entrent dans la composition de panneaux photovoltaïques. Ces utilisations sont également des objets de l'invention. En particulier, l'invention a pour objet un procédé de fabrication d'un dispositif électrochimique, de préférence une batterie, ledit procédé comprenant une étape de préparation d'un composé sulfonimide ou de ses sels selon le procédé décrit ci-dessus, et une étape de fabrication du dispositif électrochimique dans lequel le composé sulfonimide ou de ses sels est employé comme électrolyte.

De manière avantageuse, les dérivés de formule Ea-SO₃⁻Q⁺ (III) obtenus selon l'invention peuvent être mis en oeuvre pour la préparation de dérivés fluorés d'acide sulfonique Ea-SO₃H.

Ainsi, selon encore un autre de ses aspects, l'invention a pour objet un procédé de préparation d'un dérivé fluoré d'acide sulfonique de formule (V)

Ea-SO₃H (V)

Ea représentant l'atome de fluor ou un groupe ayant de 1 à 10 atomes de carbone choisi parmi les fluoroalkyles, les perfluoroalkyles et les fluoroalkényles ; en particulier Ea représentant le radical CF₃ ;
comprenant au moins les étapes suivantes :
- préparation selon le procédé décrit précédemment d'un composé de formule Ea-SO₃⁻Q⁺ (III), dans laquelle Q⁺ représente un cation onium ; et
- acidification du composé de formule (III) pour obtenir le dérivé fluoré d'acide sulfonique de formule (V) souhaité.

En particulier, un dérivé fluoré d'acide sulfonique de formule Ea-SO₃H (V) où Ea est tel que défini précédemment, peut être préparé selon l'invention *via* au moins les étapes suivantes :
(a) mise en contact d'un composé liquide ionique de formule Ea-COO⁻Q⁺ (I) à l'état liquide où Q représente un cation onium, avec un oxyde de soufre, pour former un composé de formule Ea-SOO⁻Q⁺ (II), ledit composé liquide ionique de formule (I) représentant au moins 50 % en poids du milieu réactionnel liquide initial ;
   la réaction de sulfination étant conduite jusqu'à un taux de conversion dudit composé de formule (I) allant de 10 à 90 %, en particulier de 30 à 70 % et plus particulièrement de 50 à 60 % ;
(b) ajout au mélange réactionnel liquide (M) des composés de formule Ea-COO⁻Q⁺ (I) et Ea-SOO⁻Q⁺ (II), obtenu à l'issue de l'étape (a) de sulfination, d'un agent oxydant, pour former un composé de formule Ea-SO₃⁻Q⁺ (III) ; et
(c2) acidification pour obtenir le dérivé fluoré d'acide sulfonique de formule (V) souhaité.

Les étapes de sulfination (a) et d'oxydation (b) sont plus particulièrement opérées dans les conditions décrites précédemment.

L'acidification du composé de formule Ea-SO₃⁻Q⁺ (III) peut être opérée par ajout d'acide sulfurique, en particulier sous forme d'oléum.

Plus particulièrement, l'acidification du mélange des composés de formule Ea-SO₃⁻Q⁺ et Ea-COO⁻Q⁺, obtenu à l'issue de l'oxydation, conduit au mélange du dérivé fluoré d'acide sulfonique Ea-SO₃H souhaité et à l'acide fluorocarboxylique Ea-COOH, par exemple au mélange d'acide triflique et d'acide trifluoroacétique (dans le cas particulier où Ea représente CF₃).

Le dérivé fluoré d'acide sulfonique Ea-SO₃H peut être isolé du mélange obtenu à l'issue de l'acidification, par exemple par distillation.

Le dérivé fluoré d'acide carboxylique Ea-COOH est avantageusement recyclé, par exemple dans le procédé selon l'invention.

Avantageusement, le procédé de l'invention est mis en oeuvre pour préparer l'acide trifluorométhanesulfonique CF₃SO₃H, plus couramment appelé acide triflique.

Selon un mode de réalisation particulier, le composé de formule (I) mis en oeuvre en étape (a) est un sel d'onium d'acide trifluorocarboxylique, en particulier le trifluorocarboxylate de tétrabutylphosphonium (CF₃COOPBu₄), et conduit, à l'issue de l'étape (c2), à l'acide triflique (CF₃SO₃H).

Le dérivé fluoré d'acide sulfonique Ea-SO₃H obtenu selon l'invention peut être avantageusement transformé en anhydride de formule (Ea-SO₂)₂O (VI).

Ainsi, selon encore un autre de ses aspects, l'invention a pour objet un procédé de préparation d'un composé anhydride de formule (Ea-SO₂)₂O (VI), Ea représentant l'atome de fluor ou un groupe ayant de 1 à 10 atomes de carbone choisi parmi les fluoroalkyles, les perfluoroalkyles et les fluoroalkényles, en particulier Ea représentant le radical CF₃ ;
comprenant au moins les étapes suivantes :
- préparation selon le procédé décrit précédemment d'un dérivé fluoré d'acide sulfonique de formule Ea-SO₃H (V) ; et
- anhydrisation du dérivé de formule Ea-SO₃H (V) pour obtenir ledit composé anhydride de formule (VI) souhaité.

En particulier, un composé anhydride de formule (Ea-SO₂)₂O (VI), où Ea est tel que défini précédemment, peut être préparé selon l'invention *via* au moins les étapes suivantes :
(a) mise en contact d'un composé liquide ionique de formule Ea-COO⁻Q⁺ (I) à l'état liquide où Q représente un cation onium, avec un oxyde de soufre, pour former un composé de formule Ea-SOO⁻Q⁺ (II), ledit composé liquide ionique de formule (I) représentant au moins 50 % en poids du milieu réactionnel liquide initial ;
   la réaction de sulfination étant conduite jusqu'à un taux de conversion dudit composé de formule (I) allant de 10 à 90 %, en particulier de 30 à 70 % et plus particulièrement de 50 à 60 % ;
(b) ajout au mélange réactionnel liquide (M) des composés de formule Ea-COO⁻Q⁺ (I) et Ea-SOO⁻Q⁺ (II), obtenu à l'issue de l'étape (a) de sulfination, d'un agent oxydant, pour former un composé de formule Ea-SO₃⁻Q⁺ (III) ;
(c2) acidification pour obtenir le dérivé fluoré d'acide sulfonique de formule Ea-SO₃H (V) ; et
(d3) anhydrisation du composé de formule (V) pour former le composé anhydride de formule (VI) souhaité.

Les étapes de sulfination (a), d'oxydation (b) et d'acidification (c2) sont plus particulièrement opérées dans les conditions décrites précédemment.

La réaction d'anhydrisation est connue de l'homme du métier et est plus particulièrement décrite dans le document US 8,222,450.

Avantageusement, le procédé de l'invention est mis en oeuvre pour préparer l'anhydride trifluorométhanesulfonique (CF₃-SO₂)₂O, plus couramment appelé anhydride triflique.

Selon un mode de réalisation particulier, le composé de formule (I) mis en oeuvre en étape (a) est un sel d'onium d'acide trifluorocarboxylique, en particulier le trifluorocarboxylate de tétrabutylphosphonium (CF₃COOPBu₄), et conduit, à l'issue de l'étape (d3), à l'anhydride triflique ((CF₃-SO₂)₂O).

Les dérivés fluorés d'acide sulfonique de formule Ea-SO₃H, notamment l'acide triflique, et les composés anhydrides de formule (Ea-SO₂)₂O, notamment l'anhydride triflique, peuvent être utilisés dans diverses applications, notamment comme catalyseur acide, comme groupe de protection en synthèse organique, comme synthon dans les domaines de la pharmaceutique, l'agrochimie ou l'électronique, comme sel pour l'électronique, ou comme composant d'un liquide ionique.

L'invention va maintenant être décrite au moyen des exemples suivant donnés bien entendu à titre illustratif et non limitatif de l'invention.

### EXEMPLES

Le taux de conversion d'un réactif correspond au rapport entre la quantité molaire de réactif consommée (transformée) au cours d'une réaction sur la quantité de réactif initiale.

Le rendement en produit à partir d'un réactif correspond au rapport de la quantité molaire de produit formé sur la quantité molaire de réactif initial.

Le bilan pondéral correspond au rapport du poids total de matière récupérée sur le poids total de matière engagée.

### 1. Préparation du TFAPBu₄ (CF₃CO₂PBu₄)

Dans un réacteur en verre de 0,25L, sont introduits :
- TFAK (CF₃COOK) : 43,8g (∼0.3 mol)
- PBu₄Cl : 86,7g (∼0.3 mol)
- H₂O :110g

La masse réactionnelle est portée à 50 °C sous agitation pendant 20 heures. Après refroidissement et arrêt de l'agitation, le milieu décante en deux phases liquides, qui sont séparées et analysées ; la phase supérieure est essentiellement constituée de CF₃CO₂PBu₄ et d'eau (7 % en poids).

La réaction de formation du TFAPBu₄ peut être représentée comme suit :

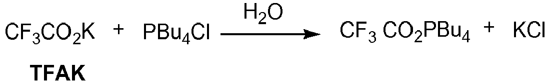

### 2. Sulfination du TFAPBu₄ en TFSPBu₄ (CF₃SO₂PBu₄)

Dans un autoclave de 0,1 mL, préalablement lavé et séché, sont introduits :
- TFAPBu₄: 49,2 g, soit 0,12 mol (préalablement séché par distillation azéotropique),
- SO₂ : 7,76 g, soit SO₂/TFAPBu₄ 103 % mol.

Le réacteur est ensuite fermé et chauffé sous agitation à 142 °C pendant 4h.

Après retour à l'ambiante (20-22 °C), le réacteur est dégazé, et son contenu est transféré dans une recette en verre de 0,1 L ; le milieu réactionnel résultant se présente sous la forme d'une solution brun foncé.

La réaction de sulfination du TFAPBu₄ en TFSPBu₄ peut être représentée comme suit :

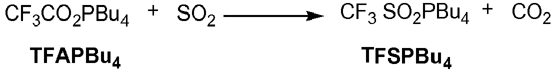

L'analyse par ¹⁹F RMN de la masse réactionnelle conduit aux résultats suivants :
- Bilan pondéral : 94 %
- Conversion du TFAPBu₄ : 61 %
- Rendement en TFSPBu₄ : 38 %

### 3. Oxydation du triflinate CF₃SO₂PBu₄ (TFSPBu₄) en triflate CF₃SO₃PBu₄ (TAPBu₄)

Le brut réactionnel de l'étape 2. précédente (45 g) est chargé dans un ballon tricol de 0,1 L et porté à 80 °C ; l'eau oxygénée (solution aqueuse 30 % : 6 g) est coulée en 3 heures sur le milieu réactionnel maintenu à 80 °C.

Après coulée, le milieu réactionnel est maintenu à 80 °C pendant 2 heures.

Après retour à l'ambiante (20-22 °C), le milieu réactionnel résultant se présente sous la forme d'une solution brun clair.

La réaction d'oxydation du triflinate en triflate peut être représentée comme suit :

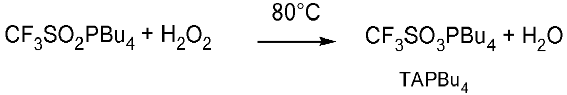

L'analyse par ¹⁹F RMN de la masse réactionnelle conduit aux résultats suivants :
- Bilan pondéral : 100 %
- Conversion du TFSPBu₄ : 100 %
- Rendement en TAPBu₄ : 97 %
NB : conversion du TFAPBu₄ résiduel : 0 %

### 4. Acidification/Distillation

Dans un réacteur en verre de 50 ml sont introduits :
- brut réactionnel précédent : 50 g
- oléum 20 % : 122 g

Sous agitation, le milieu est porté progressivement à 160 °C sous vide progressif (pression finale : 5mbar) et les condensats sont recueillis et analysés pour conduire aux résultats suivants :
- Taux de récupération du TFA (CF₃COOH) : 65 %
- Taux de récupération du TA (CF₃SO₃H) : 50 %

## Revendications

1. Procédé de préparation d'un dérivé oxysulfuré et fluoré sous forme de sel de formule (II)
Ea-SOO⁻Q⁺ (II)
comprenant la mise en présence d'un composé liquide ionique de formule (I) à l'état liquide
Ea-COO⁻Q⁺ (I)
- Ea représentant l'atome de fluor ou un groupe ayant de 1 à 10 atomes de carbone choisi parmi les fluoroalkyles, les perfluoroalkyles et les fluoroalkényles ; et
- Q⁺ représentant un cation onium,
avec un oxyde de soufre, ledit composé liquide ionique de formule (I) représentant au moins 50 % en poids du milieu réactionnel liquide initial.

2. Procédé selon la revendication 1, dans lequel le milieu réactionnel est exempt de solvant organique de type amide tel que le N,N-diméthylformamide (DMF), la N-méthylpyrrolidone (NMP) ou le N,N-diméthylacétamide (DMAC).

3. Procédé selon la revendication 1 ou la revendication 2, dans lequel le cation onium Q⁺ est choisi parmi les cations ammonium, phosphonium, pyridinium, pyrazolinium, imidazolium, arsénium, ammonium quaternaire et phosphonium quaternaire, de préférence parmi les cations tétraalkylphosphonium et tétraalkylammonium dont les groupes alkyle, identiques ou différents, représentent une chaîne alkyle linéaire ou ramifiée ayant de 4 à 12 atomes de carbone, de préférence de 4 à 6 atomes de carbone, et les cations tétraarylphosphonium et tétraarylammonium dont les groupes aryles, identiques ou différents, représentent un groupe phényle ou naphtalényle.

4. Procédé selon l'une quelconque des revendications 1 à 3, dans lequel le cation onium Q⁺ est un cation phosphonium quaternaire, de préférence le cation tétrabutylphosphonium (PBu₄).

5. Procédé selon l'une quelconque des revendications 1 à 3, dans lequel le cation Q⁺ représente un cation ammonium quaternaire, en particulier choisi dans le groupe constitué par le tétraéthylammonium, le tétrapropylammonium, le tétrabutylammonium, le triméthylbenzylammonium, le méthyltributylammonium.

6. Procédé selon l'une quelconque des revendications 1 à 3, dans lequel le cation Q⁺ représente un cation pyridinium, en particulier le N-méthylpyridinium.

7. Procédé selon l'une quelconque des revendications 1 à 6, pour la préparation d'un sel d'onium d'acide trifluorométhanesulfinique CF₃SOO⁻Q⁺, en particulier du trifluorométhane sulfinate de tétrabutylphosphonium CF₃SOOPBu₄.

8. Procédé de préparation d'un composé sous forme de sel de formule (III)
Ea-SO₃⁻Q⁺ (III)
- Ea représentant l'atome de fluor ou un groupe ayant de 1 à 10 atomes de carbone choisi parmi les fluoroalkyles, les perfluoroalkyles et les fluoroalkényles ; et
- Q⁺ représentant un cation onium,
comprenant les étapes consistant en :
(i) disposer d'un mélange (M), présentant une température de fusion inférieure ou égale à 100 °C, en particulier inférieure ou égale à 40 °C et plus particulièrement inférieure ou égale à 20 °C, d'un composé liquide ionique de formule Ea-COO⁻Q⁺ (I) et d'un composé de formule Ea-SOO⁻Q⁺ (II) ; et
(ii) mettre en présence ledit mélange (M) à l'état liquide avec un agent oxydant, pour obtenir le composé de formule Ea-SO₃⁻Q⁺ (III), ledit mélange (M) représentant plus de 50 % en poids du milieu réactionnel liquide initial.

9. Procédé selon la revendication 8, pour la préparation d'un sel d'onium d'acide trifluorométhanesulfonique CF₃SO₃⁻Q⁺, en particulier du trifluorométhanesulfonate de tétrabutylphosphonium CF₃SO₃PBu₄.

10. Procédé de préparation d'un composé sulfonimide de formule (Ea-SO₂)₂NH (IV) ou un de ses sels (Ea-SO₂)₂NMe (IV'),
Ea représentant l'atome de fluor ou un groupe ayant de 1 à 10 atomes de carbone choisi parmi les fluoroalkyles, les perfluoroalkyles et les fluoroalkényles ; et
Me représentant un métal alcalin, en particulier le lithium ;
comprenant au moins les étapes suivantes :
(a1) préparation d'un dérivé oxysulfuré et fluoré de formule Ea-SO₂⁻Q⁺ (II) selon le procédé décrit dans l'une quelconque des revendications 1 à 7 ;
(b1) bromation, chloration ou fluoration du dérivé de formule (II) pour former un composé de formule (Ea-SO₂)X avec X représentant le brome, le chlore ou le fluor ;
(c1) ammonolyse du composé (Ea-SO₂)X à l'aide d'une amine tertiaire NR"₃ en (EaSO₂)₂NH.NR"₃, avec R", identiques ou différents, représentant un groupe alkyle, linéaire ou ramifié, ayant de 1 à 20 atomes de carbone ;
(d1) acidification de (EaSO₂)₂NH.NR"₃ pour obtenir le composé sulfonimide (Ea-SO₂)₂NH (IV) ;
et éventuellement :
(e1) neutralisation, par une base de métal alcalin Me, en particulier par un hydroxyde alcalin, du composé (Ea-SO₂)₂NH pour former le sel de formule (Ea-SO₂)₂NMe (IV') ; et éventuellement
(f1) séchage du sel (Ea-SO₂)₂NMe (IV').

11. Procédé selon la revendication 10 pour la préparation du bis-(trifluorométhanesulfonyl)imide (CF₃SO₂)₂NH et du bis-(trifluorométhanesulfonyl)imidure de lithium (LiTFSI).

12. Procédé de préparation d'un dérivé fluoré d'acide sulfonique de formule (V)
Ea-SO₃H (V)
Ea représentant l'atome de fluor ou un groupe ayant de 1 à 10 atomes de carbone choisi parmi les fluoroalkyles, les perfluoroalkyles et les fluoroalkényles ;
comprenant au moins les étapes suivantes :
- préparation selon le procédé tel que défini l'une quelconque des revendications 8 ou 9 d'un dérivé de formule Ea-SO₃⁻Q⁺ (III), dans laquelle Q⁺ représente un cation onium ; et
- acidification du composé de formule (III) pour obtenir le dérivé fluoré d'acide sulfonique de formule (V) souhaité.

13. Procédé selon la revendication 12, pour la préparation de l'acide trifluorométhane sulfonique CF₃SO₃H.

14. Procédé de préparation d'un composé anhydride de formule (VI)
(Ea-SO₂)₂O (VI)
Ea représentant l'atome de fluor ou un groupe ayant de 1 à 10 atomes de carbone choisi parmi les fluoroalkyles, les perfluoroalkyles et les fluoroalkényles ; en particulier Ea représentant le radical CF₃ ;
comprenant au moins les étapes suivantes :
- préparation selon le procédé tel que défini selon l'une la revendication 12 ou la revendication 13 d'un dérivé fluoré d'acide sulfonique de formule Ea-SO₃H ; et
- anhydrisation du dérivé de formule Ea-SO₃H pour obtenir ledit composé anhydride de formule (VI) souhaité.

15. Procédé selon la revendication 14 pour la préparation de l'anhydride trifluorométhanesulfonique (CF₃-SO₂)₂O.

## Patentansprüche

1. Verfahren zur Herstellung eines Fluoroxysulfid-Derivats in Form eines Salzes der Formel (II)
Ea-SOO⁻Q⁺ (II),
bei dem man eine ionische flüssige Verbindung der Formel (I) in flüssigem Zustand
Ea-COO⁻Q⁺ (I),
- wobei Ea für ein Fluoratom oder eine Gruppe mit 1 bis 10 Kohlenstoffatomen, die aus Fluoralkylgruppen, Perfluoralkylgruppen und Fluoralkenylgruppen ausgewählt ist, steht und
- wobei Q⁺ für ein Onium-Kation steht, mit einem Schwefeloxid zusammenbringt, wobei die ionische flüssige Verbindung der Formel (I) mindestens 50 Gew.-% des anfänglichen flüssigen Reaktionsmediums ausmacht.

2. Verfahren nach Anspruch 1, bei dem das Reaktionsmedium frei von organischem Lösungsmittel vom Amid-Typ wie N,N-Dimethylformamid (DMF), N-Methylpyrrolidon (NMP) oder N,N-Dimethylacetamid (DMAC) ist.

3. Verfahren nach Anspruch 1 oder Anspruch 2, bei dem das Onium-Kation Q⁺ aus Ammonium-, Phosphonium-, Pyridinium-, Pyrazolinium-, Imidazolium-, Arsenium-, quaternären Ammonium- und quaternären Phosphonium-Kationen, vorzugsweise aus Tetraalkylphosphonium- und Tetraalkylammonium-Kationen, deren Alkylgruppen, die gleich oder verschieden sind, eine lineare oder verzweigte Alkylkette mit 4 bis 12 Kohlenstoffatomen und vorzugsweise 4 bis 6 Kohlenstoffatomen darstellen, und Tetraarylphosphonium- und Tetraarylammonium-Kationen, deren Arylgruppen, die gleich oder verschieden sind, eine Phenyl- oder Naphthalinylgruppe darstellen, ausgewählt ist.

4. Verfahren nach einem der Ansprüche 1 bis 3, wobei sich bei dem Onium-Kation Q⁺ um ein quaternäres Phosphonium-Kation, vorzugsweise das Tetrabutylphosphonium(PBu₄)-Kation, handelt.

5. Verfahren nach einem der Ansprüche 1 bis 3, bei dem das Kation Q⁺ ein quaternäres Ammonium-Kation, insbesondere aus der Gruppe bestehend aus Tetraethylammonium, Tetrapropylammonium, Tetrabutylammonium, Trimethylbenzylammonium und Methyltributylammonium, darstellt.

6. Verfahren nach einem der Ansprüche 1 bis 3, dem das Kation Q⁺ ein Pyridinium-Kation, insbesondere N-Methylpyridinium, darstellt.

7. Verfahren nach einem der Ansprüche 1 bis 6 zur Herstellung eines Trifluormethansulfinsäure-Oniumsalzes CF₃SOO⁻Q⁺, insbesondere von Tetrabutylphosphoniumtrifluormethansulfinat CF₃SOOPBu₄.

8. Verfahren zur Herstellung einer Verbindung in Form eines Salzes der Formel (III)
Ea-SO₃⁻Q⁺ (III),
- wobei Ea für ein Fluoratom oder eine Gruppe mit 1 bis 10 Kohlenstoffatomen, die aus Fluoralkylgruppen, Perfluoralkylgruppen und Fluoralkenylgruppen ausgewählt ist, steht und
- wobei Q⁺ für ein Onium-Kation steht,
bei dem man:
(i) eine einen Schmelzpunkt kleiner oder gleich 100 °C, insbesondere kleiner oder gleich 40 °C und spezieller kleiner oder gleich 20 °C aufweisende Mischung (M) einer ionischen flüssigen Verbindung der Formel Ea-COO⁻Q⁺ (I) und einer Verbindung der Formel Ea-SOO⁻Q⁺ (II) bereitstellt und
(ii) die Mischung (M) in flüssigem Zustand mit einem Oxidationsmittel zusammenbringt, was die Verbindung der Formel Ea-SO₃⁻Q⁺ (III) ergibt, wobei die Mischung (M) mehr als 50 Gew.-% des anfänglichen flüssigen Reaktionsmediums ausmacht.

9. Verfahren nach Anspruch 8 zur Herstellung eines Trifluormethansulfonsäure-Oniumsalzes CF₃SO₃⁻Q⁺, insbesondere von Tetrabutylphosphoniumtrifluormethansulfonat CF₃SO₃PBu₄.

10. Verfahren zur Herstellung einer Sulfonimid-Verbindung der Formel (Ea-SO₂)₂NH (IV) oder eines ihrer Salze (Ea-SO₂)₂NMe (IV'),
wobei Ea für ein Fluoratom oder eine Gruppe mit 1 bis 10 Kohlenstoffatomen, die aus Fluoralkylgruppen, Perfluoralkylgruppen und Fluoralkenylgruppen ausgewählt ist, steht und
wobei Me für ein Alkalimetall, insbesondere Lithium, steht;
bei dem man:
(a1) nach dem in einem der Ansprüche 1 bis 7 beschriebenen Verfahren ein Fluoroxysulfid-Derivat der Formel Ea-SO₂⁻Q⁺ (II) herstellt;
(b1) das Derivat der Formel (II) bromiert, chloriert oder fluoriert, was eine Verbindung der Formel (Ea-SO₂)X ergibt, wobei X für Brom, Chlor oder Fluor steht;
(c1) die Verbindung (Ea-SO₂)X mit einem tertiären Amin NR"₃ zu (Ea-SO₂)₂NH.NR"₃ ammonolysiert, wobei R" gleich oder verschieden ist und für eine lineare oder verzweigte Alkylgruppe mit 1 bis 20 Kohlenstoffatomen steht;
(d1) (Ea-SO₂)₂NH.NR"₃ ansäuert, was die Sulfonimid-Verbindung (Ea-SO₂)₂NH (IV) ergibt; und gegebenenfalls:
(e1) die Verbindung (Ea-SO₂)₂NH mit einer Alkalimetall-Me-Base, insbesondere mit einem Alkalimetallhydroxid, neutralisiert, was das Salz der Formel (Ea-SO₂)₂NMe (IV') ergibt; und gegebenenfalls
(f1) das Salz (Ea-SO₂)₂NMe (IV') trocknet.

11. Verfahren nach Anspruch 10 zur Herstellung von Bis(trifluormethansulfonyl)imid (CF₃SO₂)₂NH und Lithiumbis(trifluormethansulfonyl)imid (LiTFSI).

12. Verfahren zur Herstellung eines fluorierten Sulfonsäurederivats der Formel (V)
Ea-SO₃H (V),
wobei Ea für ein Fluoratom oder eine Gruppe mit 1 bis 10 Kohlenstoffatomen, die aus Fluoralkylgruppen, Perfluoralkylgruppen und Fluoralkenylgruppen ausgewählt ist, steht;
bei dem man:
- nach dem Verfahren gemäß einem der Ansprüche 8 oder 9 ein Derivat der Formel Ea-SO₃⁻Q⁺ (III), worin Q⁺ für ein Onium-Kation steht, herstellt und
- die Verbindung der Formel (III) ansäuert, was das gewünschte fluorierte Sulfonsäurederivat der Formel (V) ergibt.

13. Verfahren nach Anspruch 12 zur Herstellung von Trifluormethansulfonsäure CF₃SO₃H.

14. Verfahren zur Herstellung einer Anhydridverbindung der Formel (VI)
(Ea-SO₂)₂O (VI),
wobei Ea für ein Fluoratom oder eine Gruppe mit 1 bis 10 Kohlenstoffatomen, die aus Fluoralkylgruppen, Perfluoralkylgruppen und Fluoralkenylgruppen ausgewählt ist, steht und Ea insbesondere für den CF₃-Rest steht;
bei dem man:
- nach dem Verfahren gemäß Anspruch 12 oder Anspruch 13 ein fluoriertes Sulfonsäurederivat der Formel Ea-SO₃H herstellt und
- das Derivat der Formel Ea-SO₃H anhydrisiert, was die gewünschte Anhydridverbindung der Formel (VI) ergibt.

15. Verfahren nach Anspruch 14 zur Herstellung von Trifluormethansulfonsäureanhydrid (CF₃-SO₂)₂O.

## Claims

1. Process for the preparation of an oxysulfide and fluorinated derivative in the form of a salt of formula (II):
Ea-SOO⁻Q⁺ (II)
comprising the operation in which an ionic liquid compound of formula (I) in the liquid state:
Ea-COO⁻Q⁺ (I)
- Ea representing a fluorine atom or a group having from 1 to 10 carbon atoms selected from fluoroalkyls, perfluoroalkyls and fluoroalkenyls; and
- Q⁺ representing an onium cation,
is brought together with a sulfur oxide, said ionic liquid compound of formula (I) representing at least 50% by weight of the initial liquid reaction medium.

2. Process according to Claim 1, in which the reaction medium is devoid of organic solvent of amide type, such as N,N-dimethylformamide (DMF), N-methylpyrrolidone (NMP) or N,N-dimethylacetamide (DMAC).

3. Process according to Claim 1 or Claim 2, in which the onium cation Q⁺ is selected from ammonium, phosphonium, pyridinium, pyrazolinium, imidazolium, arsenium, quaternary ammonium and quaternary phosphonium cations, preferably from tetraalkylphosphonium and tetraalkylammonium cations, the alkyl groups of which, which are identical or different, represent a linear or branched alkyl chain having from 4 to 12 carbon atoms, preferably from 4 to 6 carbon atoms, and tetraarylphosphonium and tetraarylammonium cations, the aryl groups of which, which are identical or different, represent a phenyl or naphthyl group.

4. Process according to any one of Claims 1 to 3, in which the onium cation Q⁺ is a quaternary phosphonium cation, preferably the tetrabutylphosphonium (PBu₄) cation.

5. Process according to any one of Claims 1 to 3, in which the cation Q⁺ represents a quaternary ammonium cation selected in particular from the group consisting of tetraethylammonium, tetrapropylammonium, tetrabutylammonium, trimethylbenzylammonium and methyltributylammonium.

6. Process according to any one of Claims 1 to 3, in which the cation Q⁺ represents a pyridinium cation, in particular N-methylpyridinium.

7. Process according to any one of Claims 1 to 6, for the preparation of a trifluoromethanesulfinic acid onium salt CF₃SOO⁻Q⁺, in particular tetrabutylphosphonium trifluoromethanesulfinate CF₃SOOPBu₄.

8. Process for the preparation of a compound in the form of a salt of formula (III):
Ea-SO₃⁻Q⁺ (III)
- Ea representing a fluorine atom or a group having from 1 to 10 carbon atoms selected from fluoroalkyls, perfluoroalkyls and fluoroalkenyls; and
- Q⁺ representing an onium cation,
comprising the stages consisting in:
(i) having available a mixture (M), exhibiting a melting point of less than or equal to 100°C, in particular of less than or equal to 40°C and more particularly of less than or equal to 20°C, of an ionic liquid compound of formula Ea-COO⁻Q⁺ (I) and of a compound of formula Ea-SOO⁻Q⁺ (II); and
(ii) bringing together said mixture (M) in the liquid state and an oxidizing agent, in order to obtain the compound of formula Ea-SO₃⁻Q⁺ (III), said mixture (M) representing more than 50% by weight of the initial liquid reaction medium.

9. Process according to Claim 8, for the preparation of a trifluoromethanesulfonic acid onium salt CF₃SO₃⁻Q⁺, in particular tetrabutylphosphonium trifluoromethanesulfonate CF₃SO₃PBu₄.

10. Process for the preparation of a sulfonimide compound of formula (Ea-SO₂)₂NH (IV) or one of its salts (Ea-SO₂)₂NMe (IV'),
Ea representing a fluorine atom or a group having from 1 to 10 carbon atoms selected from fluoroalkyls, perfluoroalkyls and fluoroalkenyls; and
Me representing an alkali metal, in particular lithium;
comprising at least the following stages:
(a1) preparation of an oxysulfide and fluorinated derivative of formula Ea-SO₂⁻Q⁺ (II) according to the process described in any one of Claims 1 to 7;
(b1) bromination, chlorination or fluorination of the derivative of formula (II) in order to form a compound of formula (Ea-SO₂)X, with X representing bromine, chlorine or fluorine;
(c1) ammonolysis of the compound (Ea-SO₂)X using a tertiary amine NR"₃ to give (EaSO₂)₂NH.NR"₃, with R", which are identical or different, representing a linear or branched alkyl group having from 1 to 20 carbon atoms;
(d1) acidification of (EaSO₂)₂NH.NR"₃ to obtain the sulfonimide compound (Ea-SO₂)₂NH (IV) ;
and optionally:
(e1) neutralization by an alkali metal Me base, in particular by an alkali metal hydroxide, of the compound (Ea-SO₂)₂NH in order to form the salt of formula (Ea-SO₂)₂NMe (IV'); and optionally
(f1) drying of the salt (Ea-SO₂)₂NMe (IV').

11. Process according to Claim 10, for the preparation of bis(trifluoromethanesulfonyl)imide (CF₃SO₂)₂NH and of lithium bis(trifluoromethanesulfonyl)imide (LiTFSI).

12. Process for the preparation of a fluorinated derivative of sulfonic acid of formula (V):
Ea-SO₃H (V)
Ea representing a fluorine atom or a group having from 1 to 10 carbon atoms selected from fluoroalkyls, perfluoroalkyls and fluoroalkenyls;
comprising at least the following stages:
- preparation according to the process as defined in either one of Claims 8 and 9 of a derivative of formula Ea-SO₃⁻Q⁺ (III), in which Q⁺ represents an onium cation; and
- acidification of the compound of formula (III) in order to obtain the desired fluorinated derivative of sulfonic acid of formula (V).

13. Process according to Claim 12, for the preparation of trifluoromethanesulfonic acid CF₃SO₃H.

14. Process for the preparation of an anhydride compound of formula (VI):
(Ea-SO₂)₂O (VI)
Ea representing a fluorine atom or a group having from 1 to 10 carbon atoms selected from fluoroalkyls, perfluoroalkyls and fluoroalkenyls, in particular Ea representing the CF₃ radical;
comprising at least the following stages:
- preparation according to the process as defined according to Claim 12 or Claim 13 of a fluorinated derivative of sulfonic acid of formula Ea-SO₃H; and
- anhydrization of the derivative of formula Ea-SO₃H in order to obtain said desired anhydride compound of formula (VI).

15. Process according to Claim 14, for the preparation of trifluoromethanesulfonic anhydride (CF₃-SO₂)₂O.
